# EUROPÄISCHE PATENTANMELDUNG

(11) **EP 4 785 965 A1**
(43) Veröffentlichungstag der Anmeldung: **05.08.2026**
(21) Anmeldenummer: 25154963.0
(22) Anmeldetag: 30.01.2025
(51) Int. Cl.: A61L 9/03, A61L 9/12, A61L 9/14

(54) **ANORDNUNG UMFASSEND EIN AN EINEN STECKDOSENEINSATZ ANKOPPELBARES FUNKTIONSMODUL**

(71) Anmelder: Albrecht Jung GmbH & Co. KG, 58579 Schalksmühle (DE)
(72) Erfinder: Rauch, Daniel, 42115 Wuppertal (DE)

(57) **Zusammenfassung**

Vorgestellt wird eine Anordnung umfassend einen in einer Dose angeordneten Steckdoseneinsatz E und ein als Unterputz-Diffusoraufsatz zum Ankoppeln an den Steckdoseneinsatz E ausgeführtes Funktionsmodul F, wobei der Unterputz-Diffusoraufsatz einen Tank T mit einem flüssigen Duftmittel, einen Zerstäuber zum Zerstäuben des flüssigen Duftmittels und einen elektrischen Anschluss zum Anschluss an eine Energieversorgung umfasst, wobei der elektrische Anschluss des Unterputz-Diffusoraufsatzes als ein kabelloser, an die Rückseite (R) des Unterputz-Diffusoraufsatzes angeformter Stecker CS zum Einstecken in den Steckdoseneinsatz E ausgeführt ist, wobei der Unterputz-Diffusoraufsatz an dem Steckdoseneinsatz E mittels Festlegungsmitteln festgelegt ist, und wobei der Tank T und/oder der Zerstäuber unterputz angeordnet sind, wobei der Tank T und/oder der Zerstäuber insbesondere in eine zylindrische Vertiefung V des Steckdoseneinsatzes E eingreifen.

## Beschreibung

Die Erfindung betrifft eine Anordnung umfassend einen in einer Dose angeordneten Steckdoseneinsatz und ein als Unterputz-Diffusoraufsatz zum Ankoppeln an den Steckdoseneinsatz ausgeführtes Funktionsmodul.

Die Erfindung betrifft ferner ein Verfahren zum Zerstäuben eines flüssigen Duftmittels mit einer solchen Anordnung.

Funktionsmodule, die dazu dienen, den Funktionsumfang von Installationsgeräten der Gebäudeinstallationstechnik zu erweitern, sind beispielsweise aus DE 10 2023 133 998 B3 oder DE 10 2023 110 987 B3 bekannt. Dabei handelt es sich typischerweise um Dimmer, Jalousieaktoren, Schaltaktoren oder dergleichen. Reizvoll dabei ist, dass die Hürde für die Nachrüstung weiterer Funktionen insofern abgesenkt ist, als dass keine vollständige Neuinstallation erforderlich ist, sondern die Funktionsmodule regelmäßig nachrüstbar sind.

Ein im Rahmen der Gebäudeinstallationstechnik eher selten bearbeitetes Gebiet betrifft Funktionsmodule, die dazu geeignet sind, die Raumluft zu beduften.

Bekannt sind Neuinstallationen, die in Installationsdosen installiert werden, beispielsweise aus EP 4 157 370 A1, und zur Raumluftverbesserung dienen. Diese sind insofern aufwendig, als dass sie die Installation einer Installationsdose mit entsprechender Verkabelung erfordern, so dass die Hürde für eine Nachrüstung hoch liegt, wobei regelmäßig ein Elektriker involviert wird.

Ebenfalls sind Duftdiffusoren bekannt, die via Plug-and-Play mittels Stecker in eine Steckdose, insbesondere in eine zylindrische Vertiefung mit zwei Steckdosenöffnungen, einsteckbar sind. Diese Lösungen sind jedoch weder ästhetisch noch kompakt, da sie ein Netzteil erfordern und bauraum ineffizient ausgeführt sind. Dadurch erhalten diese in eine Steckdose einsteckbare Duftdiffusoren eine ausladende Geometrie, so dass sie regelmäßig 10 cm und mehr von der Steckdose abstehen. Damit entsteht das Problem, dass die Duftdiffusoren nicht nur wackelig anmuten, sondern auch eine Stolperfalle für Personen darstellen beziehungsweise durch Kollision leicht beschädigt werden können.

Ausgehend von diesem diskutierten Stand der Technik liegt der Erfindung die Aufgabe zugrunde, eine Anordnung mit einem Diffusor bereitzustellen, die nicht nur kompakt ist und sich in ein Installationsgeräteprogramm der Gebäudeinstallationstechnik einfügt, sondern darüber hinaus auch im Wege der Nachrüstung ohne das Herstellen einer neuen Installationsdose und aufwendiger Verkabelung montierbar ist.

Gelöst wird diese Aufgabe durch eine Anordnung umfassend einen in einer Dose angeordneten Steckdoseneinsatz und ein als Unterputz-Diffusoraufsatz zum Ankoppeln an den Steckdoseneinsatz ausgeführtes Funktionsmodul, wobei der Unterputz-Diffusoraufsatz einen Tank mit einem flüssigen Duftmittel, einen Zerstäuber zum Zerstäuben des flüssigen Duftmittels und einen elektrischen Anschluss zum Anschluss an eine Energieversorgung umfasst, wobei der elektrische Anschluss des Unterputz-Diffusoraufsatzes als ein kabelloser, an die Rückseite des Unterputz-Diffusoraufsatzes angeformter Stecker zum Einstecken in den Steckdoseneinsatz ausgeführt ist, wobei der Unterputz-Diffusoraufsatz an dem Steckdoseneinsatz mittels Festlegungsmitteln festgelegt ist, und wobei der Tank und/oder der Zerstäuber unterputz angeordnet sind, wobei der Tank und/oder der Zerstäuber insbesondere in eine zylindrische Vertiefung des Steckdoseneinsatzes eingreifen.

Der erste besonders vorteilhafte Aspekt der erfindungsgemäßen Anordnung liegt darin begründet, dass eine Installation einer Installationsdose inklusive entsprechender Verkabelungstätigkeiten vermieden wird, indem die erfindungsgemäße Anordnung ein als Unterputz-Diffusoraufsatz zum Ankoppeln an den Steckdoseneinsatz ausgeführtes Funktionsmodul aufweist. Dabei ist der elektrische Anschluss des Unterputz-Diffusoraufsatzes als ein kabelloser, an die Rückseite des Unterputz-Diffusoraufsatzes angeformter Stecker zum Einstecken in den Steckdoseneinsatz ausgeführt.

Der zweite Vorteil der erfindungsgemäßen Anordnung ist darin zu finden, dass im Gegensatz zu vorbekannten, in eine Steckdose einsetzbaren Diffusoren die erfindungsgemäße Anordnung lediglich von einem Steckdoseneinsatz ausgeht, so dass eine Zentralplatte vorteilhafterweise entbehrlich wird. Hierdurch wird wertvoller Einbauraum eingespart, der dem Tankvolumen zugutekommt.

Daran anknüpfend wird bereits der dritte Vorteil der erfindungsgemäßen Anordnung sichtbar: Auf geschickte Art und Weise wird der Diffusoraufsatz, im Gegensatz zum Stand der Technik, als Unterputz-Diffusoraufsatz ausgeführt, indem der Tank und/oder der Zerstäuber unterputz angeordnet sind, wobei der Tank und/oder der Zerstäuber insbesondere in eine zylindrische Vertiefung des Steckdoseneinsatzes eingreifen. Mithin wird eine besonders ästhetisch anmutende Kompaktheit ermöglicht.

Der Unterputz-Diffusoraufsatz weist einen Tank mit einem flüssigen Duftmittel, einen Zerstäuber zum Zerstäuben des flüssigen Duftmittels und einen elektrischen Anschluss zum Anschluss an eine Energieversorgung auf. Dabei ist der Unterputz-Diffusoraufsatz an dem Steckdoseneinsatz mittels Festlegungsmitteln festgelegt, wodurch ein vierter Vorteil der erfindungsgemäßen Anordnung begründet wird, der darin besteht, dass der erfindungsgemäße Unterputz-Diffusoraufsatz im Gegensatz zu vorbekannten Einsteck-Lösungen, bei denen ein Stecker in eine Steckdose eingesetzt - mithin nicht festgelegt - wird, ein Wackeln wirksam vermieden wird.

Gemäß einer Weiterbildung der erfindungsgemäßen Anordnung weist der Stecker mindestens zwei Steckerstifte zum Einführen in zwei Steckdosensockelöffnungen des Steckdoseneinsatzes auf und ist beispielsweise als Euro- oder Schukostecker ausgeführt.

In einer alternativen Weiterbildung der erfindungsgemäßen Anordnung ist der Stecker zum Einstecken in eine einen USB-Anschluss aufweisende USB-Steckdose ausgeführt, wobei der Stecker als USB-Stecker ausgeführt ist. Dabei wird der Vorteil ausgenutzt, dass eine USB-Steckdose bereits ein Netzteil aufweist, so dass eine besonders attraktive Kompaktheit der erfindungsgemäßen Anordnung erreicht wird.

Gemäß einer Weiterbildung der erfindungsgemäßen Anordnung ist der Tank des Unterputz-Diffusoraufsatzes umlaufend von einem Designrahmen des Steckdoseneinsatzes umgeben. Dieses erhöht den Grad der optischen Integration in ein Installationsgeräteprogramm und erhöht zudem die mechanische Stabilität bezüglich etwaiger Kollisionen, so dass der Unterputz-Diffusoraufsatz sich im Bedarfsfall an dem Designrahmen abstützt beziehungsweise der Designrahmen etwaige seitliche mechanische Einwirkungen, wie Stöße, effektiv abfängt beziehungsweise abschwächt.

Gemäß einer Weiterbildung der erfindungsgemäßen Anordnung weisen die Festlegungsmittel einen Halteabsatz auf, der zum Einsetzen in eine zylindrische Vertiefung des Steckdoseneinsatzes geeignet ist. Dabei umfasst der Halteabsatz elastische Elemente, die die zylindrische Vertiefung jedenfalls abschnittsweise umlaufend kontaktieren, um die Haltekraft des Unterputz-Diffusoraufsatzes an der zylindrischen Vertiefung des Steckdoseneinsatzes zu erhöhen. Optional ist dabei der Halteabsatz in eine radiale Richtung der zylindrischen Vertiefung werkzeuglos vergrößerbar ausgeführt, um die Haltekraft noch weiter zu erhöhen.

In einer Weiterbildung der erfindungsgemäßen Anordnung weist der Unterputz-Diffusoraufsatz Einstellmittel zur Einstellung der Diffusorleistung, insbesondere via Applikation auf einem Endgerät oder mittels Betätigungselement, auf. Damit kann die Diffusorleistung bequem eingestellt und sogar automatisiert werden, beispielsweise nach Raumgröße oder typischen Nutzungsintervallen, so dass der Verbrauch an Duftmittel reduzierbar ist.

Gemäß einer Weiterbildung der erfindungsgemäßen Anordnung weist der Unterputz-Diffusoraufsatz eine Erhebung im zentralen Bereich auf, die von seinem Rand beabstandet ist, so dass die Höhe des Unterputz-Diffusoraufsatzes vom Rand aus zunimmt. Hierdurch wird nicht nur das Tankvolumen vergrößert, sondern eine optisch schlanke Erscheinung gesichert.

Gemäß einer Weiterbildung der erfindungsgemäßen Anordnung erzeugt der Zerstäuber eine ventilatorbasierte Diffusion, eine heizbasierte Verdampfung, eine Kaltluftzerstäubung oder einen Ultraschall-Nebel.

Gemäß einer bevorzugten Weiterbildung der erfindungsgemäßen Anordnung ist der Tank so ausgebildet, dass er mit einem Abschnitt tiefer in die Dose eintaucht als der Boden der zylindrischen Vertiefung. Hierdurch wird auf geschickte Weise das in Anspruch genommene Volumen für den Tank noch weiter vergrößert, indem ungenutzte Freiräume eines typischen Steckdoseneinsatzes beansprucht werden.

In einer Weiterbildung der erfindungsgemäßen Anordnung zeigt der Diffusor mittels einer optischen Anzeige den Betriebszustand des Unterputz-Diffusoraufsatzes an. So kann einem Anwender beispielsweise die Leistung des Diffusors angezeigt werden. In einer bevorzugten Weiterbildung der erfindungsgemäßen Anordnung ist der Tank transparent ausgeführt, um eine optische Füllstandsanzeige zu ermöglichen. Somit erkennt der Anwender unmittelbar, wann der Tank aufzufüllen ist, so dass ein Leerlaufen des Tanks wirksam vermieden ist. Bevorzugt ist der Tank und/oder das Funktionsmodul mechanisch reversibel an den Steckdoseneinsatz gekoppelt, um das Befüllen oder Ersetzen (falls der Tank als Kartusche ausgeführt ist) des Tanks zu erleichtern. Zum Befüllen kann die erfindungsgemäße Anordnung auch um ein Abfüllwerkzeug, insbesondere einen Trichter, erweitert werden.

Gemäß einer bevorzugten Weiterbildung der erfindungsgemäßen Anordnung, die sich chemisch besonders resistent gegenüber typischen Duftmitteln zeigt, ist der Tank aus Glas oder Keramik ausgeführt. Damit kann eine besonders hohe Konzentration des Duftmittels gewählt und eine kompakte Bauform gewährleistet werden, ohne dass der Tank chemisch angegriffen wird und seine Lebensdauer in Mitleidenschaft gezogen wird.

Die Erfindung betrifft zudem ein Verfahren zum Zerstäuben eines flüssigen Duftmittels mit der erfindungsgemäßen Anordnung umfassend die Schritte:
- Einstecken des Unterputz-Diffusoraufsatzes in den Steckdoseneinsatz,
- Befestigen des Unterputz-Diffusoraufsatzes mittels Festlegungsmitteln an dem Steckdoseneinsatz,
- Starten des Zerstäubens des flüssigen Duftmittels.

Nachfolgend ist die Erfindung unter Bezugnahme auf die beigefügten Figuren anhand von sechs Ausführungsbeispielen beschrieben. Es zeigen:
- **Fig. 1:**: eine schematische Explosionsdarstellung der erfindungsgemäßen Anordnung in einer perspektivischen Vorderansicht,
- **Fig. 2:**: eine schematische Explosionsdarstellung der erfindungsgemäßen Anordnung gemäß Figur 1 in einer perspektivischen Rückansicht,
- **Fig. 3:**: eine schematische Darstellung der erfindungsgemäßen Anordnung gemäß Figur 1 in einer perspektivischen Vorder- beziehungsweise Rückansicht,
- **Fig. 4:**: eine schematische Explosionsdarstellung einer weiteren erfindungsgemäßen Anordnung in einer perspektivischen Vorderansicht,
- **Fig. 5:**: eine schematische Explosionsdarstellung der erfindungsgemäßen Anordnung gemäß Figur 4 in einer perspektivischen Rückansicht, und
- **Fig. 6:**: eine schematische Darstellung der erfindungsgemäßen Anordnung gemäß Figur 4 in einer perspektivischen Vorder- beziehungsweise Rückansicht.

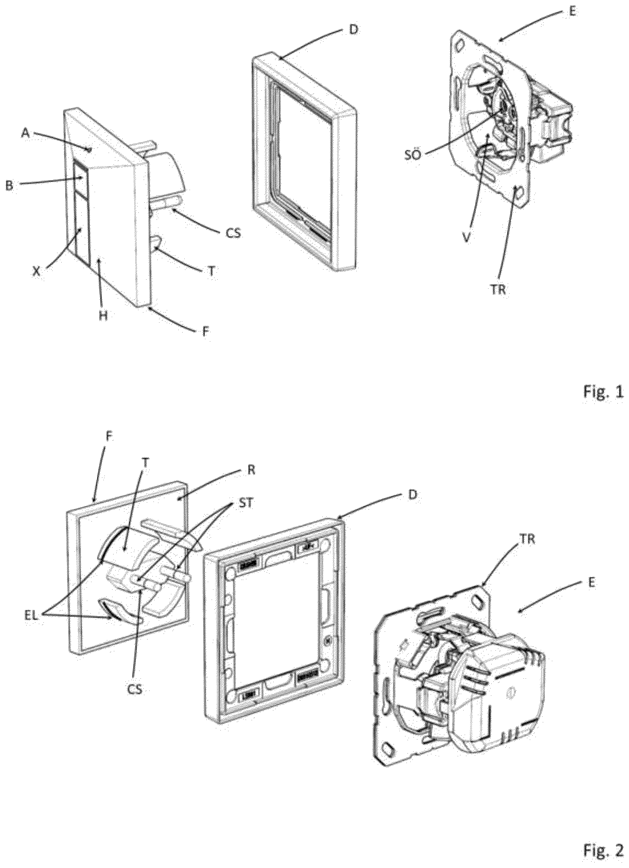

Figur 1 zeigt eine schematische Explosionsdarstellung der erfindungsgemäßen Anordnung in einer perspektivischen Vorderansicht. Dabei umfasst die erfindungsgemäße Anordnung einen in einer Dose (der Übersichtlichkeit halber nicht dargestellt) angeordneten Steckdoseneinsatz E und ein als Unterputz-Diffusoraufsatz zum Ankoppeln an den Steckdoseneinsatz E ausgeführtes Funktionsmodul F, wobei der Unterputz-Diffusoraufsatz einen Tank T mit einem flüssigen Duftmittel, einen Zerstäuber zum Zerstäuben des flüssigen Duftmittels und einen elektrischen Anschluss zum Anschluss an eine Energieversorgung umfasst. Der elektrische Anschluss des Unterputz-Diffusoraufsatzes ist als ein kabelloser, an die Rückseite R des Unterputz-Diffusoraufsatzes angeformter Stecker CS (hier ein Schukostecker) zum Einstecken in den Steckdoseneinsatz E ausgeführt, wobei der Unterputz-Diffusoraufsatz an dem Steckdoseneinsatz E, der regelmäßig mittels Tragring TR an einer Dose festgelegt ist, mittels Festlegungsmitteln festgelegt ist und wobei der Tank T und/oder der Zerstäuber unterputz angeordnet sind, wobei der Tank T und/oder der Zerstäuber insbesondere in eine zylindrische Vertiefung V des Steckdoseneinsatzes E eingreifen. Vorliegend umfasst der Stecker CS mindestens zwei Steckerstifte ST zum Einführen in zwei Steckdosensockelöffnungen SÖ des Steckdoseneinsatzes E und ist als Eurostecker ausgeführt. Die erfindungsgemäße Anordnung erlaubt eine verkabelungsfreie Nachrüstung einer Steckdose durch einen Unterputz-Diffusoraufsatz. Dabei kommt die erfindungsgemäße Anordnung intelligenterweise gerade ohne die regelmäßig vorhandene Zentralplatte einer Steckdose aus, sondern verwendet stattdessen das Funktionsmodul F beziehungsweise den Unterputz-Diffusoraufsatz. Unter dem Begriff unterputz wird im Sinne der Erfindung der Bereich verstanden, der in einer Dose, regelmäßig einer Installationsdose, angeordnet ist; mithin in einer Wand, einer Fliese, einem Möbel oder dergleichen liegt.

Figur 2 zeigt eine schematische Explosionsdarstellung der erfindungsgemäßen Anordnung gemäß Figur 1 in einer perspektivischen Rückansicht. Dabei ist eine Ausführungsform gezeigt, wobei die Festlegungsmittel einen Halteabsatz aufweisen, der zum Einsetzen in eine zylindrische Vertiefung V der Steckdose geeignet ist, wobei der Halteabsatz elastische Elemente EL aufweist, die die zylindrische Vertiefung V, beispielsweise den Tragring TR, jedenfalls abschnittsweise umlaufend kontaktieren, um die Haltekraft des Unterputz-Diffusoraufsatzes an der zylindrischen Vertiefung V des Steckdoseneinsatzes E zu erhöhen. Alternativ können die Festlegungsmittel auch als Verrastungsmittel des Funktionsmoduls F mit dem Tragring TR ausgeführt sein. Dabei können die Verrastungsmittel ein gewisses Maß an Halte- beziehungsweise Abzugskraft des Funktionsmoduls F am Steckdoseneinsatz E bereitstellen, bei deren Überschreitung das Funktionsmodul F erst entfernbar ist.

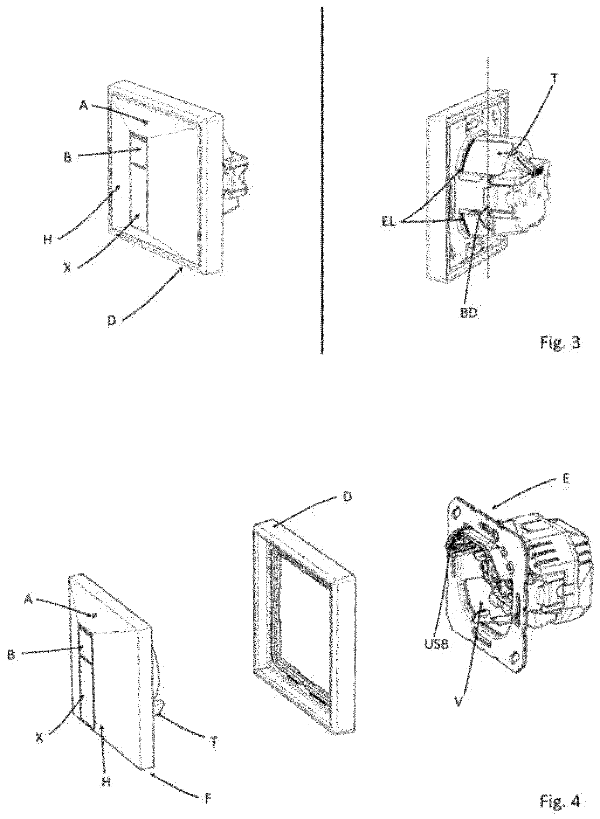

Figur 3 zeigt eine schematische Darstellung der erfindungsgemäßen Anordnung gemäß Figur 1 in einer perspektivischen Vorder- beziehungsweise Rückansicht (links beziehungsweise rechts). Dabei weist der Unterputz-Diffusoraufsatz eine Erhebung H im zentralen Bereich auf, die von seinem Rand beabstandet ist, so dass die Höhe des Unterputz-Diffusoraufsatzes vom Rand aus zunimmt. Besonders effizient, um das Volumen des Tanks T zu maximieren, ist die Ausbildung des Tanks T, dass er mit einem Abschnitt tiefer in die Dose eintaucht als der Boden BD (die gepunktete Vertikale deutet die Bodenebene an) der zylindrischen Vertiefung V. Hierdurch werden Freiräume einer typischen Steckdose geschickt hinsichtlich des Tankvolumens nutzbar gemacht. Ferner ist der Tank T transparent ausgeführt, um eine optische Füllstandsanzeige X zu ermöglichen. Zudem zeigt Figur 3 ein Betätigungselement B sowie einen Auslass A für das durch den Zerstäuber zerstäubte Duftmittel.

Figur 4 zeigt eine schematische Explosionsdarstellung einer weiteren erfindungsgemäßen Anordnung in einer perspektivischen Vorderansicht, wobei der Stecker CS zum Einstecken in einen, einen USB-Anschluss USB aufweisende, USB-Steckdoseneinsatz ausgeführt ist, wobei der Stecker CS als USB-Stecker ausgeführt ist. In einer solchen Ausführungsform ergibt sich der Vorteil, dass ein Netzteil, das typischerweise die Spannung auf ein geringeres Maß heruntertransformiert, bereits in den Steckdoseneinsatz E integriert ist, so dass der USB-Steckdoseneinsatz bezüglich eines regulären Steckdoseneinsatzes E weitergebildet ist. Hierdurch wird weiterer Bauraum eingespart.

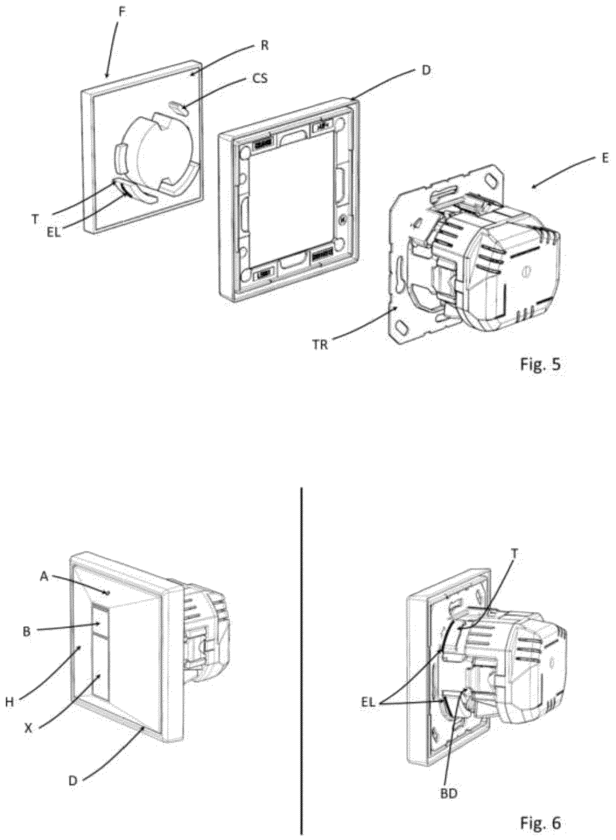

Figur 5 zeigt eine schematische Explosionsdarstellung der erfindungsgemäßen Anordnung gemäß Figur 4 in einer perspektivischen Rückansicht, wobei erkennbar ist, dass der Stecker CS als USB-Stecker ausgeführt ist. Ein solcher Stecker CS ist regelmäßig kleiner als ein Schuko- oder Eurostecker, so dass der Tank T noch größer ausgeführt werden kann und in die zylindrische Vertiefung V, vorzugsweise mit einem Volumen von mehr als 20 cm³, eingreift.

Figur 6 zeigt eine schematische Darstellung der erfindungsgemäßen Anordnung gemäß Figur 4 in einer perspektivischen Vorder- beziehungsweise Rückansicht (links beziehungsweise rechts). Dabei ist erkennbar, dass der als USB-Steckdoseneinsatz ausgeführt Steckdoseneinsatzes E aufgrund eines integrierten Netzteils voluminöser ausgebildet ist.

### Bezugszeichenliste

- A: Auslass
- B: Betätigungselement
- BD: Boden
- CS: Stecker
- D: Designrahmen
- E: Steckdoseneinsatz
- F: Funktionsmodul
- EL: elastische Elemente
- H: Erhebung
- R: Rückseite
- SÖ: Steckdosensockelöffnung
- ST: Steckerstifte
- T: Tank
- TR: Tragring
- USB: USB-Anschluss
- V: zylindrische Vertiefung
- X: Füllstandsanzeige

## Patentansprüche

1. Anordnung umfassend einen in einer Dose angeordneten Steckdoseneinsatz (E) und ein als Unterputz-Diffusoraufsatz zum Ankoppeln an den Steckdoseneinsatz (E) ausgeführtes Funktionsmodul (F), wobei der Unterputz-Diffusoraufsatz einen Tank (T) mit einem flüssigen Duftmittel, einen Zerstäuber zum Zerstäuben des flüssigen Duftmittels und einen elektrischen Anschluss zum Anschluss an eine Energieversorgung umfasst, wobei der elektrische Anschluss des Unterputz-Diffusoraufsatzes als ein kabelloser, an die Rückseite (R) des Unterputz-Diffusoraufsatzes angeformter Stecker (CS) zum Einstecken in den Steckdoseneinsatz (E) ausgeführt ist, wobei der Unterputz-Diffusoraufsatz an dem Steckdoseneinsatz (E) mittels Festlegungsmitteln festgelegt ist und wobei der Tank (T) und/oder der Zerstäuber unterputz angeordnet sind, wobei der Tank (T) und/oder der Zerstäuber insbesondere in eine zylindrische Vertiefung (V) des Steckdoseneinsatzes (E) eingreifen.

2. Anordnung nach Anspruch 1, **dadurch gekennzeichnet, dass** der Stecker (CS) mindestens zwei Steckerstifte (ST) zum Einführen in zwei Steckdosensockelöffnungen (SÖ) des Steckdoseneinsatzes (E) umfasst und beispielsweise als Euro- oder Schukostecker ausgeführt ist.

3. Anordnung nach Anspruch 1 oder 2, **dadurch gekennzeichnet, dass** der Tank (T) des Unterputz-Diffusoraufsatzes umlaufend von einem Designrahmen (D) des Steckdoseneinsatzes (E) umgeben ist.

4. Anordnung nach einem der Ansprüche 1 bis 3, **dadurch gekennzeichnet, dass** die Festlegungsmittel einen Halteabsatz aufweisen, der zum Einsetzen in eine zylindrische Vertiefung (V) des Steckdoseneinsatzes (E) geeignet ist, wobei der Halteabsatz elastische Elemente (EL) aufweist, die die zylindrische Vertiefung (V) jedenfalls abschnittsweise umlaufend kontaktieren, um die Haltekraft des Unterputz-Diffusoraufsatzes an der zylindrischen Vertiefung (V) des Steckdoseneinsatzes (E) zu erhöhen.

5. Anordnung nach einem der Ansprüche 1 bis 4, **dadurch gekennzeichnet, dass** die Festlegungsmittel als Verrastungsmittel des Funktionsmoduls (F) mit einem Tragring (TR) ausgeführt sind.

6. Anordnung nach einem der Ansprüche 3 bis 5, **dadurch gekennzeichnet, dass** der Stecker (CS) zum Einstecken in einen, einen USB-Anschluss (USB) aufweisende, USB-Steckdoseneinsatz ausgeführt ist, wobei der Stecker (CS) als USB-Stecker ausgeführt ist.

7. Anordnung nach einem der Ansprüche 1 bis 6, **dadurch gekennzeichnet, dass** der Unterputz-Diffusoraufsatz Einstellmittel zur Einstellung der Diffusorleistung, insbesondere via Applikation auf einem Endgerät oder mittels Betätigungselement (B), aufweist.

8. Anordnung nach einem der Ansprüche 1 bis 7, **dadurch gekennzeichnet, dass** der Unterputz-Diffusoraufsatz eine Erhebung (H) im zentralen Bereich aufweist, die von seinem Rand beabstandet ist, so dass die Höhe des Unterputz-Diffusoraufsatzes vom Rand aus zunimmt.

9. Anordnung nach einem der Ansprüche 1 bis 8, **dadurch gekennzeichnet, dass** der Zerstäuber eine ventilatorbasierte Diffusion, eine heizbasierte Verdampfung, eine Kaltluftzerstäubung oder einen Ultraschall-Nebel erzeugt.

10. Anordnung nach einem der Ansprüche 1 bis 9, **dadurch gekennzeichnet, dass** der Tank (T) so ausgebildet ist, dass er mit einem Abschnitt tiefer in die Dose eintaucht als der Boden (BD) der zylindrischen Vertiefung.

11. Anordnung nach einem der Ansprüche 1 bis 10, **dadurch gekennzeichnet, dass** der Unterputz-Diffusoraufsatz eine optische Anzeige aufweist, die den Betriebszustand des Unterputz-Diffusoraufsatzes anzeigt.

12. Anordnung nach einem der Ansprüche 1 bis 11, **dadurch gekennzeichnet, dass** der Tank (T) transparent ausgeführt ist, um eine optische Füllstandsanzeige (X) zu ermöglichen.

13. Anordnung nach einem der Ansprüche 1 bis 12, **dadurch gekennzeichnet, dass** der Tank (T) aus Glas oder Keramik ausgeführt ist.

14. Verfahren zum Zerstäuben eines flüssigen Duftmittels mit der Anordnung gemäß einem der Ansprüche 1 bis 13 umfassend die Schritte:
- Einstecken des Unterputz-Diffusoraufsatzes in den Steckdoseneinsatz (E),
- Befestigen des Unterputz-Diffusoraufsatzes mittels Festlegungsmitteln an dem Steckdoseneinsatz (E),
- Starten des Zerstäubens des flüssigen Duftmittels.
